# FASCICULE DE BREVET EUROPEEN

(11) **EP 0 818 201 B1**
(45) Date de publication et mention de la délivrance du brevet: **13.03.2002**
(21) Numéro de dépôt: 97401609.9
(22) Date de dépôt: 04.07.1997
(51) Int. Cl.: A61K 31/715

(54) **Utilisation d'un polyholoside pour stimuler les défenses immunitaires**
Verwendung eines Polysaccharids zur Stimulierung der Immunabwehr
Use of a polysaccharide to stimulate immune defenses

(30) Priorité: 10.07.1996 FR 9608617
(43) Date de publication de la demande: 14.01.1998
(73) Titulaire: L'OREAL, 75008 Paris (FR)
(72) Inventeur: Breton, Lionel, 78000 Versailles (FR); Desolle, Pierre, 92310 Sevres (FR); Pineau, Nathalie, 86000 Poitiers (FR)
(74) Mandataire: Tezier Herman, Béatrice

(56) Documents cités:
- EP-A- 0 344 955
- EP-B- 0 053 800
- DE-A- 4 221 753
- H. WAGNER ET AL.: "Immunstimulierend wirkende Polysaccharide (Heteroglykane) aus höheren Pflanzen." DRUG RESEARCH, vol. 35, no. 7, 1985, pages 1069-1075, XP000646167
- C. ET R. DUVAL: "Dictionnaire de la chimie et de ses applications", 1978, TECHNIQUE ET DOCUMENTATION, PARIS

## Description

L'invention concerne l'utilisation en tant que composé actif dans une composition cosmétique ou pour la préparation d'une composition pharmaceutique, en particulier dermatologique, d'une quantité efficace d'au moins un polyholoside, ce polyholoside ou la composition étant destiné à stimuler les défenses immunitaires. Elle se rapporte aussi à l'utilisation d'au moins un polyholoside à titre de médicament, ainsi qu'à des compositions comprenant au moins un polyholoside.

Le système immunitaire comprend un ensemble de cellules spécialisées soumises à de multiples mécanismes de contrôle assurant leur renouvellement, leur activation et leur différenciation, indispensables à un niveau normal d'immunocompétence. Le rôle du système immunitaire est de discriminer le soi du non soi pour éliminer les agents pathogènes et les tumeurs spontanées. Toute déplétion cellulaire, toute dysrégulation immunitaire ou tout déficit fonctionnel est susceptible de favoriser la survenue de manifestations pathologiques caractérisées par la perturbation des mécanismes de reconnaissance du soi vis-à-vis du non soi, et une plus grande sensibilité vis-à-vis des agressions microbiennes et des processus néoplasiques.

La peau constitue l'organe le plus important de l'organisme et est reconnue comme l'un des principaux éléments actifs du système de défense immunitaire. Trois types de cellules épidermiques participent à ce système : les kératinocytes, les mélanocytes et les cellules de Langerhans. Ces cellules que l'on ne retrouve qu'au niveau de la peau, jouent un rôle primordial dans la réponse immunitaire et en particulier dans la présentation antigénique.

La peau saine est capable de se défendre des agressions extérieures grâce aux moyens mis à sa disposition. Néanmoins, elle est soumise à l'agression permanente de l'environnement, de produits chimiques, de radiations. En particulier les cellules de Langerhans sont la cible privilégiée des rayonnements ultraviolets.

Ces agressions se traduisent par un effet suppresseur des défenses immunitaires entraînant une baisse des résistances aux agents pathogènes et une augmentation de l'incidence de certains cancers.

Pour aider la peau à remplir sa fonction immunitaire, des produits de stimulation du système immunitaire cutanée sont d'un grand intérêt.

On sait d'autre part que le système immunitaire et plus particulièrement celui de la peau s'affaiblit au cours du vieillissement chronobiologique.
Cet affaiblissement survient également au cours du vieillissement photo-induit. Un effet immunostimulateur peut alors rétablir les fonctions immunitaires et plus particulièrement celles de l'épiderme en renforçant les défenses naturelles de la peau.

Un autre but de l'invention est donc de proposer un nouvel immunostimulant, plus particulièrement du système immunitaire de la peau.

La demanderesse a trouvé de manière inattendue que l'application d'une quantité efficace d'au moins un polyholoside comprenant au moins un motif fucose permet de stimuler les défenses immunitaires.

Le brevet européen EP-B-0053800 décrit un hétéroglycane composé de fucose, mannose et glucose ayant une activité immunostimulatrice.

Les saccharides, de formule Cₙ(H₂O)ₙ, sont généralement divisés en deux catégories : les oses ou sucres simples, et les osides ou association de plusieurs molécules.
Parmi les osides, on peut distinguer (1) les holosides qui sont formés uniquement de sucres et (2) les hétérosides qui sont constitués par un ou plusieurs oses et une partie non glucidique.
De plus, parmi les polyholosides, on peut encore distinguer les polyholosides homogènes qui résultent de l'association d'un même ose, et les polyholosides hétérogènes qui résultent soit de l'association d'oses différents, soit de l'association d'oses ayant la même formule chimique brute mais de configuration géométrique différente (isomères D et L par exemple), considérés également dans la présente description comme des oses différents.
C'est cette dernière catégorie, constituée des polyholosides hétérogènes, qui est plus particulièrement concernée par la présente invention.

Le polyholoside hétérogène selon l'invention est constitué uniquement de sucres et résulte de l'association d'au moins deux oses différents dont l'un au moins est un fucose.
Les polyholosides selon l'invention peuvent être constitués de 2 à 10 oses, composés couramment appelés oligoholosides, ou de plus de 10 oses, composés couramment appelés polyholosides.

L'invention a pour premier objet l'utilisation en tant que composé actif dans une composition cosmétique ou pour la préparation d'une composition pharmaceutique, en particulier dermatologique, d'une quantité efficace d'au moins un polyholoside comprenant des motifs fucose, galactose et acide galacturonique, ce polyholoside ou la composition étant destiné à stimuler les défenses immunitaires.

Un autre objet de l'invention est l'utilisation de ce polyholoside à titre de médicament.

Un autre objet de l'invention est une composition cosmétique ou pharmaceutique, comprenant, à titre de principe actif, ce polyholoside.

L'utilisation d'un polyholoside selon l'invention présente en outre d'autres avantages. Cela permet d'obtenir une composition pour la peau peu ou non irritante et peu collante, qui présente de surcroît un toucher doux et agréable.
Un autre avantage procuré par l'utilisation d'un tel polyholoside est d'améliorer la stabilisation de la composition finale, lorsqu'elle se présente sous forme d'émulsion, grâce aux propriétés auto-émulsionnantes du polyholoside employé, en particulier lorsqu'il s'agit d'un polyholoside comprenant un motif fucose.

De plus, l'incorporation d'un polyholoside dans des compositions, notamment cosmétiques, permet l'obtention d'une composition gélifiée sans ajout supplémentaire d'agent gélifiant classiquement utilisé. Le gel obtenu est lisse et onctueux.

Encore plus préférentiellement le polyholoside comprend un enchaînement linéaire d'α-L-Fucose, d' α-D-Galactose et d'acide galacturonique.

De préférence, le polyholoside hétérogène comprend au moins un motif fucose, qui peut être présent en une quantité de 10-90% en poids, de préférence 15-35% en poids, par rapport au poids de matière sèche de polyholoside.

Dans le cadre de la présente invention, on peut utiliser le polyholoside hétérogène selon l'invention seul, ou en mélange avec d'autres polyholosides hétérogènes.

Le polyholoside selon l'invention peut être éventuellement ramifié ou linéaire. Il peut également être substitué, par exemple par des chaînes grasses, notamment comprenant 8 à 30 atomes de carbone.

Le polyholoside de l'invention peut être de toute origine, naturel ou synthétique. Particulièrement, on peut utiliser selon l'invention un polyholoside préparé à partir de micro-organisme, comme par exemple *Klebsiella pneumoniae*. Encore plus particulièrement on utilise selon l'invention un polyholoside préparé à partir de la souche *Klebsiella pneumoniae subsp*. *Pneumoniae* dite BEC1000.

Lorsque l'on utilise un polyholoside d'origine naturelle, produit à partir d'un micro-organisme, il est généralement associé à des protéines. II peut alors être soumis à un traitement protéolytique.
Toute technique permettant l'hydrolyse des protéines peut alors être mise en oeuvre. On citera de préférence l'hydrolyse enzymatique.

Le polyholoside peut être présent dans la composition finale en une quantité de 10⁻⁴ à 20% en poids par rapport au poids de la composition et de préférence de 10⁻³ à 10% en poids par rapport au poids de la composition.

Le polyholoside présente de préférence une viscosité de 800-1200 mPa.s (viscosité Brookfield LV31, 12 tours/min, à 30°C) lorsqu'il est mis en solution dans l'eau à une concentration d'environ 1% en poids.

L'invention a également pour objet un polyholoside tel que défini précédement, à titre de médicament.

Selon un autre aspect, l'invention concerne une composition cosmétique ou pharmaceutique, comprenant, à titre de principe actif, au moins un polyholoside tel que défini précédement.

La composition pharmaceutique est préférentiellement une composition à usage dermatologique.

La composition pharmaceutique est particulièrement destinée à stimuler les défenses immunitaires, et encore plus particulièrement destinée à stimuler les défenses immunitaires de la peau.

Le polyholoside peut être présent dans la composition selon l'invention en une quantité de 10⁻⁴ à 20% en poids par rapport au poids de la composition et de préférence de 10⁻³ à 10% en poids, par rapport à la composition.

Les polyholosides selon l'invention peuvent donc être utilisés en tant qu'agent destiné à stimuler les défenses immunitaires, en particulier dans une composition à usage capillaire ou dans une composition pour la peau du corps et/ou du visage.

Ladite composition peut se présenter sous la forme d'une émulsion, notamment huile-dans-eau ou eau-dans-huile, voire sous la forme d'une émulsion multiple.
Elle peut également se présenter sous forme d'une solution aqueuse, éventuellement gélifiée, ou sous forme d'une lotion, par exemple biphasée, d'une crème, d'un lait, voire d'une mousse.

La composition selon l'invention peut comprendre une phase huileuse à base d'huile animale, végétale, minérale, siliconée, fluorée et/ou synthétique.
La phase huileuse peut également comprendre des alcools gras ou des acides gras, ainsi que des tensioactifs.
On peut notamment citer les huiles hydrocarbonées telles que l'huile de paraffine ou de vaseline; le perhydrosqualène; l'huile d'arara, l'huile d'amande douce, de calophyllum, de palme, de ricin, d'avocat, de jojoba, d'olive ou de germes de céréales; des alcools tels que l'alcool oléique, l'alcool linoléique ou linolénique, l'alcool isostéarique ou l'octyl dodécanol. On peut également citer les huiles siliconées telles que les PDMS, éventuellement phénylées telles que les phényltriméthicones.
La phase huileuse peut également comprendre une huile démaquillante telle qu'un ester d'acides gras, en particulier les esters obtenus à partir d'un alcool à chaîne droite ou ramifiée ayant de 1 à 17 atomes de carbone et d'un acide gras à chaîne droite ou ramifiée ayant de 3 à 18 atomes de carbone.
Un tel ester peut en particulier être choisi dans le groupe constitué par l'adipate de dioctyle, le palmitate d'éthyl-2 hexyle, l'adipate de diisopropyle, l'hexanoate d'éthyl-2 hexyle, le laurate d'éthyle, le myristate de méthyle, l'octanoate d'octyldodécyle, le néopentanoate d'isodécyle, le myristate d'éthyle, le propionate de myristyle, l'éthyl-2 hexanoate d'éthyl-2 hexyle, l'octanoate d'éthyl-2 hexyle, le caprate/caprylate d'éthyl-2 hexyle, le palmitate de méthyle, le myristate de butyle, le myristate d'isobutyle, le palmitate d'éthyle, le laurate d'isohexyle, le laurate d'hexyle, l'isostéarate d'isopropyle.
La phase huileuse peut être présente à raison de 5-95% en poids dans le cas d'une émulsion.

La composition selon l'invention peut comprendre, en outre,
. un agent permettant la mise en suspension de la phase grasse, par exemple un copolymère d'acrylates d'alkyle en C₁₀-C₃₀ et d'acide acrylique ou méthacrylique ou de leur ester (Pemulen TR1, Pemulen TR2, Carbopol 1342 de GOODRICH); ou un copolymère acrylamide/acide méthylpropane sulfonique (Sepigel de SEPPIC), et/ou
. un agent de mise en dispersion de la phase grasse, tel qu'un système émulsionnant ou vésiculaire à base de vésicules, éventuellement de taille nanométrique, constituées de lipides ioniques (liposomes) ou non ioniques, et en particulier les systèmes émulsionnants bien connus de l'homme du métier constitués de stéarate de glycéryl /PEG 100 stéarate (CTFA), d'alcool cétylique et d'alcool stéarylique.

La composition de l'invention peut comprendre, en outre, un agent pour modifier sa viscosité, et obtenir des textures plus ou moins gélifiées, tel que :
. les dérivés cellulosiques (carboxyméthylcellulose, hydroxyéthylcellulose, hydroxypropylméthylcellulose),
. les gommes naturelles telles que les gommes de xanthane, de guar, de caroube, les scléroglucanes, les dérivés de chitine ou de chitosane, les carraghénanes,
. les dérivés polycarboxyvinyliques du type Carbomer (vendues par GOODRICH sous les dénominations Carbopol, 940, 951, 980, ou par 3V-SIGMA sous la dénomination Synthalen K ou Synthalen L).

La composition selon l'invention peut également comprendre, de façon connue, des adjuvants couramment utilisés dans le domaine considéré, tels que les conservateurs, les antioxydants, les parfums, les charges telles que le kaolin ou l'amidon, voire les microsphères creuses, les pigments, les filtres UV, les séquestrants, les huiles essentielles, les matières colorantes, les actifs hydrophiles ou lipophiles tels que les hydratants, notamment la glycérine, le butylène glycol, les anti-inflammatoires comme l'allantoïne, le bisabolol, les anti-radicaux libres comme la vitamine E ou ses dérivés, les apaisants tels que l'eau de bleuet, des extraits végétaux, les dépigmentants, les actifs biologiques tels que l'urée, les acides aminés, les vitamines et leurs dérivés, les protéines, l'acide salicylique et ses dérivés, les α-hydroxy-acides, l'acide pyrrolidone carboxylique et ses sels, les céramides.
Bien entendu l'homme du métier veillera à choisir ce ou ces éventuels composés complémentaires, et/ou leur quantité, de manière telle que les propriétés avantageuses de la composition selon l'invention ne soient pas, ou substantiellement pas, altérées par l'adjonction envisagée.

La composition présente de préférence un pH qui respecte la peau, généralement compris entre 5 et 8, de préférence un pH de 5,5 à 7,5.

L'invention est illustrée plus en détail dans les exemples suivants.

### Exemple 1 : On étudie dans cet exemple, la capacité d'un polyholoside riche en fucose galactose et acide galacturonique (O.F.) à stimuler les défenses immunitaires.

Le test a été effectué sur un polyholoside préparé à partir de la souche *Klebsiella pneumoniae subsp*. *Pneumoniae,* et ayant subi un traitement protéolytique (O.F.).

Ce polyholoside d'une masse molaire inférieure à 10.000Da est obtenu en laboratoire, après hydrolyse d'une fraction de polysaccharride bactérien. Cette molécule présente notamment, les activités suivantes (activités représentatives d'une activité immunostimulante) :

| ① Etude de la stimulation des splénocytes de souris | | | | |
|---|---|---|---|---|
| Concentration en mg/ml | 0,5 | 1 | 5 | 10 |
| O.F. | 299 | 722 | 868 | 466 |

Les résultats sont exprimés en pourcentage de stimulation par rapport au témoin dont la valeur est égale à 100. Toute valeur supérieure à 200 est considérée comme positive.

Une augmentation de la prolifération des splénocytes de souris correspond à un effet stimulant du produit sur les cellules T et/ou B.

L'oligosaccharide riche en fucose galactose of acide galacturonique active la prolifération des splénocytes de souris ; c'est donc un immunostimulant potentiel.

| ② Etude de la production d'immunoglobulines par des splénocytes de souris | | | | | | |
|---|---|---|---|---|---|---|
| | IgG1 | IgG2a | IgG2b | IgG3 | IgM | IgA |
| 1 mg/ml | 209,6 | 126,5 | 107,9 | 111,1 | 105,4 | 101,2 |
| 5 mglml | 235,8 | 198,2 | 184,7 | 172,9 | 122,7 | 146 |
| 10 mg/ml | 285 | 134,1 | 205,2 | 204,9 | 124 | 145,8 |

Les résultats sont exprimés en pourcentage de stimulation par rapport au témoin dont la valeur est égale à 100.

De même que précédemment, un effet positif sur la production d'immunoglobulines signe une immunostimulation et donne un élément de réflexion supplémentaire puisqu'il précise éventuellement un effet spécifique des cellules B. Les cellules B ont un rôle très important dans la réponse immunitaire qui se traduit par :
- une production d'anticorps,
- une fonction de présentation de l'antigène.

L'oligosaccharide riche en fucose galactose et acide galacturonique présente donc une activité spécifique sur les cellules B.

En conclusion, l'ensemble des résultats présentés ci-dessus, confirme bien le rôle immunostimulant de cet oligosaccharide préparé à partir d'une fraction polysaccharidique d'origine bactérienne.

### Exemple 2 : Exemples de formulations illustrant l'invention. Ces compositions ont été obtenues par simple mélange des différents composants.

### Composition 1 : Lait pour le visage

- Huile de vaseline 7 g
- O.F.* 10 g
- Monostéarate de glycéryle, stéarate de polyéthylène glycol (100 OE) 3 g
- Polymère carboxyvinylique 0,4 g
- Alcool stéarylique 0,7 g
- Protéines de soja 3 g
- NaOH 0,4 g
- Conservateur qs
- Eau qsp 100 g

Cette composition se présente sous forme d'un lait pour le visage, ayant de bonnes propriétés cosmétiques, et étant doux et confortable à utiliser.
Le pH de la composition est d'environ 5,5.

### Composition 2 : Lotion

- O.F.* 5,00 g
- Palmitate d'éthyl-2 hexyle 10,00 g
- Cyclopentadiméthylsiloxane 20,00 g
- Butylène glycol 5,00 g
- Conservateur qs
- Eau qsp 100 g

Cette lotion, qui ne contient pas de tensioactif, favorise la desquamation de la peau.

### Composition 3 : Lait

- Palmitate d'octyle 35,00 g
- Glycérine 2,00 g
- O.F.* 0,50 g
- Polymère réticulé acrylates C10-C30/alkylacrylates 0,10 g
- Triéthanolamine 0,10 g
- Acides aminés de blé 1,00 g
- Conservateur qs
- Eau qsp 100 g

Le lait obtenu, qui ne contient pas de tensioactif, possède de bonnes propriétés cosmétiques.

### Composition 4 : Gel pour le visage

- Glycérine 10,00 g
- O.F.* 2,00 g
- Cocoamphodiacétate de disodium 1,00 g
- Conservateur qs
- Eau qsp 100 g

Le gel obtenu possède de bonnes propriétés cosmétiques.

### Composition 5 : Gel nettoyant à l'eau

- Butylène glycol 7,00 g
- Sarcosinate de lauroyl sodium 4,00 g
- O.F.* 0,04 g
- Triéthanolamine 0,80 g
- Carbomer 0,50 g
- Conservateur qs
- Eau qsp 100 g

Le gel obtenu possède de bonnes propriétés cosmétiques.

O.F.* : polyholoside comprenant du fucose, du galactose et de l'acide galacturonique préparé à partir de la souche *Klebsiella pneumoniae subsp. Pneumoniae,* et ayant subi un traitement protéolytique, vendu par la société Solabia.

## Revendications

1. Utilisation en tant que composé actif dans une composition cosmétique ou pour la préparation d'une composition pharmaceutique, d'une quantité efficace d'au moins un polyholoside comprenant des motifs fucose, galactose et acide galacturonique, ce polyholoside ou la composition étant destiné à stimuler les défenses immunitaires.

2. Utilisation selon la revendication précédente, **caractérisée en ce que** le polyholoside comprend un enchaînement linéaire de α-L-Fucose, de α-D-Galactose et d'acide galacturonique.

3. Utilisation selon l'une quelconque des revendications précédentes, **caractérisée en ce que** le polyholoside comprend un motif fucose présent en une quantité de 10-90% en poids, de préférence 15-35% en poids, par rapport au poids de matière sèche de polyholoside.

4. Utilisation selon l'une quelconque des revendications précédentes, **caractérisée en ce que** le polyholoside est substitué par des chaînes grasses, ayant de préférence 8-30 atomes de carbone.

5. Utilisation selon l'une quelconque des revendications précédentes, **caractérisée en ce que** le polyholoside est préparé à partir de micro-organismes.

6. Utilisation selon la revendication précédente, **caractérisée en ce que** le polyholoside est préparé à partir *Klebsiella pneumoniae*.

7. Utilisation selon la revendication précédente, **caractérisée en ce que** le polyholoside est préparé à partir de la souche *Klebsiella pneumoniae subsp. Pneumoniae* (dite BEC1000).

8. Utilisation selon l'une quelconque des revendications précédentes, **caractérisée en ce que** le polyholoside a préalablement été soumis un traitement protéolytique.

9. Utilisation selon l'une quelconque des revendications précédentes, **caractérisée en ce que** le polyholoside est présent dans la composition finale en une quantité de 10⁻⁴ à 20% en poids par rapport au poids de la composition et de préférence de 10⁻³ à 10% en poids par rapport au poids de la composition.

10. Utilisation selon l'une quelconque des revendications précédentes, dans une composition se présentant sous la forme d'une émulsion, d'une solution aqueuse, éventuellement gélifiée, d'une lotion notamment biphasée, d'une crème, d'un lait, d'une mousse.

11. A titre de médicament, un polyholoside tel que défini dans l'une quelconque des revendications 1 à 8.

12. Composition cosmétique ou pharmaceutique, **caractérisée en ce qu'**elle comprend, à titre de principe actif, au moins un polyholoside tel que défini dans l'une quelconque des revendications 1 à 9.

13. Composition selon la revendication 12, **caractérisée en ce que** la composition pharmaceutique est une composition à usage dermatologique.

14. Composition selon l'une quelconque des revendications 12 ou 13, **caractérisée en ce que** la composition pharmaceutique est destinée à stimuler les défenses immunitaires.

15. Composition selon l'une quelconque des revendications 12 à 14, **caractérisée en ce que** le polyholoside est présent dans la composition finale en une quantité de 10⁻⁴ à 20% en poids par rapport au poids de la composition et de préférence de 10⁻³ à 10% en poids par rapport au poids de la composition.

## Claims

1. Use as active compound in a cosmetic composition, or for the preparation of a pharmaceutical composition, of an effective quantity of at least one polyholoside comprising fucose, galactose and galacturonic acid units, this polyholoside or the composition being intended to stimulate the immune defences.

2. Use according to the preceding claim, **characterized in that** the polyholoside comprises a linear linkage of α-L-Fucose, α-D-Galactose and galacturonic acid.

3. Use according to any one of the preceding claims, **characterized in that** the polyholoside comprises a fucose unit present in a quantity of 10-90% by weight, preferably 15-35% by weight, relative to the weight of the polyholoside dry matter.

4. Use according to any one of the preceding claims, **characterized in that** the polyholoside is substituted by fatty chains having preferably 8-30 carbon atoms.

5. Use according to any one of the preceding claims, **characterized in that** the polyholoside is prepared from microorganisms.

6. Use according to the preceding claim, **characterized in that** the polyholoside is prepared from *Klebsiella pneumoniae*.

7. Use according to the preceding claim, **characterized in that** the polyholoside is prepared from the strain *Klebsiella pneumoniae subsp*. *Pneumoniae* (termed BEC 1000).

8. Use according to any one of the preceding claims, **characterized in that** the polyholoside was subjected beforehand to a proteolytic treatment.

9. Use according to any one of the preceding claims, **characterized in that** the polyholoside is present in the final composition in a quantity of 10⁻⁴ to 20% by weight relative to the weight of the composition and preferably of 10⁻³ to 10% by weight relative to the weight of the composition.

10. Use according to any one of the preceding claims, in a composition provided in the form of an emulsion, an aqueous solution which is optionally gelled, a lotion, especially a two-phase lotion, a cream, a milk, a foam.

11. As medicament, a polyholoside as defined in any one of Claims 1 to 8.

12. Cosmetic or pharmaceutical composition, **characterized in that** it comprises, as active ingredient, at least one polyholoside as defined in any one of Claims 1 to 9.

13. Composition according to Claim 12, **characterized in that** the pharmaceutical composition is a composition for dermatological use.

14. Composition according to either of Claims 12 and 13; **characterized in that** the pharmaceutical composition is intended to stimulate the immune defences.

15. Composition according to any one of Claims 12 to 14, **characterized in that** the polyholoside is present in the final composition in a quantity of 10⁻⁴ to 20% by weight relative to the weight of the composition and preferably of 10⁻³ to 10% by weight relative to the weight of the composition.

## Patentansprüche

1. Verwendung einer wirksamen Menge mindestens eines Polyholosids, das Fucose-, Galactose- und Galacturonsäureeinheiten enthält, als Wirkstoff in einer kosmetischen Zusammensetzung oder zur Herstellung einer pharmazeutischen Zusammensetzung, wobei das Polyholosid oder die Zusammensetzung zur Stimulierung der Immunabwehr vorgesehen ist.

2. Verwendung nach dem vorhergehenden Anspruch, **dadurch gekennzeichnet, daß** das Polyholosid eine gerade Kette von α-L-Fucose, α-D-Galactose und Galacturonsäure enthält.

3. Verwendung nach einem der vorhergehenden Ansprüche, **dadurch gekennzeichnet, daß** das Polyholosid die Fucoseeinheit in einer Menge von 10 bis 90 Gew.-% und vorzugsweise 15 bis 35 Gew.-%, bezogen auf die Trockensubstanz des Polyholosids, enthält.

4. Verwendung nach einem der vorhergehenden Ansprüche, **dadurch gekennzeichnet, daß** das Polyholosid mit Fettketten substituiert ist, die vorzugsweise 8 bis 30 Kohlenstoffatomen aufweisen.

5. Verwendung nach einem der vorhergehenden Ansprüche, **dadurch gekennzeichnet, daß** das Polyholosid ausgehend von Mikroorganismen hergestellt wird.

6. Verwendung nach dem vorhergehenden Anspruch, **dadurch gekennzeichnet, daß** das Polyholosid aus *Klebsiella pneumoniae* erhalten wird.

7. Verwendung nach dem vorhergehenden Anspruch, **dadurch gekennzeichnet, daß** das Polyholosid aus dem Stamm *Klebsiella pneumoniae subsp*. *Pneumoniae* (als BEC1000 bezeichnet) erhalten wird.

8. Verwendung nach einem der vorhergehenden Ansprüche, **dadurch gekennzeichnet, daß** das Polyholosid vorab proteolytisch behandelt wird.

9. Verwendung nach einem der vorhergehenden Ansprüche, **dadurch gekennzeichnet, daß** das Polyholosid in der fertigen Zusammensetzung in einer Menge von 10⁻⁴ bis 20 Gew.-%, bezogen auf das Gesamtgewicht der Zusammensetzung, und vorzugsweise 10⁻³ bis 10 Gew.-%, bezogen auf das Gesamtgewicht der Zusammensetzung, vorliegt.

10. Verwendung nach einem der vorhergehenden Ansprüche in einer Zusammensetzung, die in Form einer Emulsion, einer wäßrigen Lösung, die gegebenenfalls in ein Gel übergeführt ist, einer Lotion, insbesondere zweiphasigen Lotion, einer Creme, einer Milch oder eines Schaums vorliegt.

11. Polyholosid nach einem der Ansprüche 1 bis 8 als Arzneimittel.

12. Kosmetische oder pharmazeutische Zusammensetzung, **dadurch gekennzeichnet, daß** sie mindestens ein Polyholosid nach einem der Ansprüche 1 bis 9 als Wirkstoff enthält.

13. Zusammensetzung nach Anspruch 12, **dadurch gekennzeichnet, daß** die pharmazeutische Zusammensetzung eine Zusammensetzung zur dermatologischen Anwendung ist.

14. Zusammensetzung nach einem der Ansprüche 12 oder 13, **dadurch gekennzeichnet, daß** die pharmazeutische Zusammensetzung zur Stimulierung der Immunabwehr dienen soll.

15. Zusammensetzung nach einem der Ansprüche 12 bis 14, **dadurch gekennzeichnet, daß** das Polyholosid in der fertigen Zusammensetzung in einer Menge von 10⁻⁴ bis 20 Gew.-%, bezogen auf das Gesamtgewicht der Zusammensetzung, und vorzugsweise 10⁻³ bis 10 Gew.-%, bezogen auf das Gesamtgewicht der Zusammensetzung, vorliegt.
